# EUROPEAN PATENT SPECIFICATION

(11) **EP 3 220 991 B1**
(45) Date of publication and mention of the grant of the patent: **20.02.2019**
(21) Application number: 15801255.9
(22) Date of filing: 16.11.2015
(51) Int. Cl.: A61M 16/06

(54) **FRAME/HEADGEAR ADJUSTMENT ASSEMBLY**
RAHMEN-/KOPFBEDECKUNGSJUSTIEREINRICHTUNG
ENSEMBLE D'ADAPTATION DE CADRE/CASQUE

(30) Priority: 19.11.2014 US 201462081808 P
(43) Date of publication of application: 27.09.2017
(73) Proprietor: Koninklijke Philips N.V., 5656 AE Eindhoven (NL)
(72) Inventor: NEFF, Adam Michael, NL-5656 AE Eindhoven (NL)
(74) Representative: Freeke, Arnold
(86) International application number: PCT/IB2015/058840
(87) International publication number: WO 2016/079651

(56) References cited:
- WO-A1-2010/148453
- WO-A1-2014/025267
- WO-A1-2014/038959
- US-A1- 2014 251 337

## Description

### BACKGROUND OF THE INVENTION

### 1. Field of the Invention

The present invention relates to non-invasive ventilation and pressure support systems used to deliver a flow of breathing gas to a patient, and, in particular, to patient interface devices used in such systems that include a headgear/frame adjustment assembly.

### 2. Description of the Related Art

There are numerous situations where it is necessary or desirable to deliver a flow of breathing gas non-invasively to the airway of a patient, i.e., without intubating the patient or surgically inserting a tracheal tube in their esophagus. For example, it is known to ventilate a patient using a technique known as non-invasive ventilation. It is also known to deliver continuous positive airway pressure (CPAP) or variable airway pressure, which varies with the patient's respiratory cycle, to treat a medical disorder, such as sleep apnea syndrome, in particular, obstructive sleep apnea (OSA), or congestive heart failure.

Non-invasive ventilation and pressure support therapies involve the placement of a patient interface device including a mask component on the face of a patient. The mask component may be, without limitation, a nasal mask that covers the patient's nose, a nasal cushion having nasal prongs that are received within the patient's nares, a nasal/oral mask that covers the nose and mouth, or a full face mask that covers the patient's face. The patient interface device interfaces the ventilator or pressure support device with the airway of the patient, so that a flow of breathing gas can be delivered from the pressure/flow generating device to the airway of the patient. It is known to maintain such devices on the face of a wearer by a headgear having one or more straps adapted to fit over/around the patient's head.

Patient interface devices are designed to fit as many people in the fewest amount of variations as possible. As a result, achieving a proper fit presents challenges. Additionally, to account for the large number of people using one mask shape, adjustments are typically placed in the headgear. On some masks there are adjustments to move part of the cushion either closer to or farther from the patient's face. Because it takes time to readjust the mask, it is desirable for a patient to maintain their adjustment settings. However, certain actions, such as movement in bed or relaxation of the muscles while sleeping, can cause undesirable leaks to develop between the cushion and the patient's face. There is thus room for improvement in the area of patient interface device adjustment assemblies.

### SUMMARY OF THE INVENTION

The present invention provides a patient interface device for a pressure support system according to claim 1. The pressure support system includes a fluid coupling conduit and a gas flow generator coupled to the fluid coupling conduit. The gas flow generator produces a flow of breathing gas for a patient. The patient interface device comprises a frame member structured to be secured to the patient; and an adjustment assembly comprising: a body member coupled to the frame member, the body member being structured to be fluidly coupled to the fluid coupling conduit, and a dial member coupled to each of the frame member and the body member. When the frame member is under tension and oriented concavely with respect to the dial member, the adjustment assembly is structured to move between a first position and a second position. The frame member exerts a force on the dial member. When the adjustment assembly moves from the first position to the second position, the force increases.

US2014/0251337 discloses a patient interface comprising a frame member structured to be secured to the patient. The frame member has a first arm portion and a second arm portion. The patient interface further comprises an adjustment assembly comprising a body member coupled to the frame member. The body member is structured to be fluidly coupled to a fluid coupling conduit. A gas flow generator is coupled to the fluid coupling conduit; the gas flow generator being structured to produce a flow of breathing gas for a patient. The dial member is coupled to each of the frame member and the body member. The adjustment assembly is structured to rotate between a first position and a second position.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

### BRIEF DESCRIPTION OF THE DRAWINGS

FIG. 1 is a schematic diagram of a pressure support system according to one particular, non-limiting embodiment in which the present invention may be implemented;
FIG. 2A is a front isometric view of a patient interface device for the pressure support system of FIG. 1;
FIG. 2B is an exploded front isometric view of the patient interface device of FIG. 2A;
FIGS. 3A-3C are different views of a body member for the patient interface device of FIG. 2A
FIGS. 4A-4E are different views of a dial member for the patient interface device of FIG. 2A;
FIG. 5A is a front isometric view of the patient interface device of FIG 2A, shown with the adjustment assembly in the first position;
FIG. 5B is a front isometric view of the dial member of the patient interface device of FIG. 5A;
FIG. 5C is a rear isometric view of the adjustment assembly of the patient interface device of FIG. 5A, shown with a portion of the body member removed to see hidden structures;
FIG. 5D is a back elevation view of the patient interface device of FIG. 5A, shown without the body member;
FIG. 6A is a front isometric view of the patient interface device of FIG 2A, modified to show the adjustment assembly in the second position;
FIG. 6B is a front isometric view of the dial member of the patient interface device of FIG. 6A;
FIG. 6C is a rear isometric view of the adjustment assembly of the patient interface device of FIG. 6A, shown with a portion of the body member removed to see hidden structures;
FIG. 6D is a back elevation view of the patient interface device of FIG. 6A, shown without the body member;
FIGS. 7A and 7B are front isometric and top views, respectively, of another patient interface device, shown with the adjustment assembly in the second position, in accordance with an alternative embodiment of the disclosed concept;
FIG 7C is a top view of a frame member for the patient interface device of FIGS. 7A and 7B;
FIG. 8A is a front isometric view of another patient interface device, shown with the adjustment assembly in the second position, in accordance with an alternative embodiment of the disclosed concept;
FIG. 8B is a back elevation view of the patient interface device of FIG. 8A, shown without the body member;
FIG. 8C is a back elevation of a frame member of the patient interface device of FIGS. 8A and 8B;
FIG. 9A is a front isometric view of another patient interface device, shown with the adjustment assembly in the second position, in accordance with an alternative embodiment of the disclosed concept; and
FIG. 9B is a back elevation view of the patient interface device of FIG. 9A, shown without the body member.

### DETAILED DESCRIPTION OF EXEMPLARY EMBODIMENTS

As employed, herein, the statement that two or more parts or components are "coupled" together shall mean that the parts are joined or operate together either directly or through one or more intermediate parts or components. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

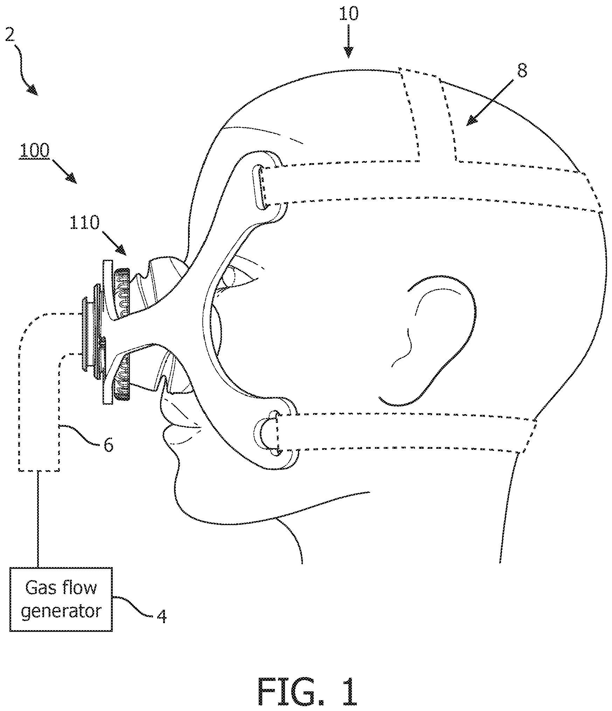

FIG. 1 shows a pressure support system 2 in accordance with the disclosed concept. Pressure support system 2 includes a gas flow generator 4 (shown in simplified form) coupled to a fluid coupling conduit (e.g., without limitation, hose 6, shown in simplified form). In operation, gas flow generator 4 produces a flow of breathing gas for a patient 10. Pressure support system 2 further includes a patient interface device 100 and headgear straps 8 that secure patient interface device 100 to patient 10. As will be discussed in greater detail below, patient interface device 100 advantageously includes an adjustment assembly 110 that allows patient 10 to quickly and easily minimize leaks that arise during pressure support therapy while maintaining the same adjustment settings.

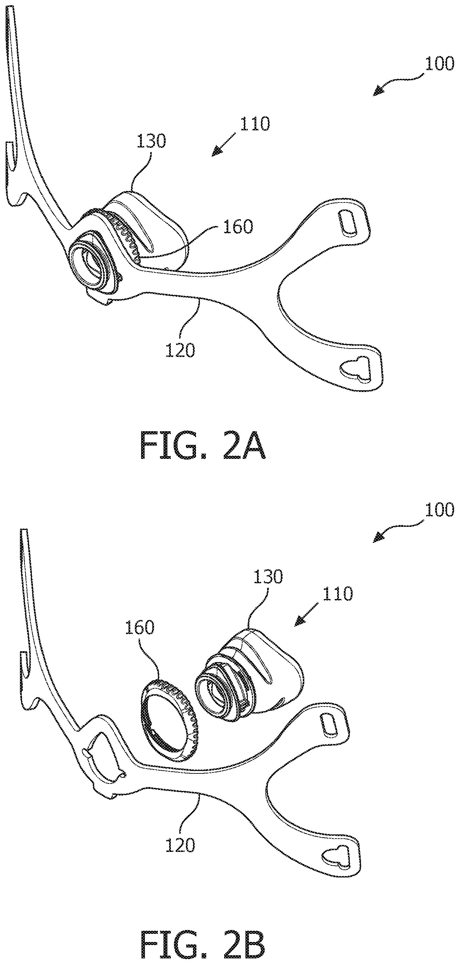

FIGS. 2A and 2B show isometric and exploded isometric views, respectively, of patient interface device 100. As shown, patient interface device 100 further includes a frame member 120 that is secured to patient 10 (FIG. 1). Adjustment assembly 110 includes a body member 130 coupled to frame member 120 and fluidly coupled to hose 6 (FIG. 1). Adjustment assembly 110 also includes a dial member 160 coupled to frame member 120 and body member 130. Body member 130 extends through frame member 120 and dial member 160. In operation (i.e., when patient interface device 100 is secured to patient 10 (FIG. 1) and gas flow generator 4 (FIG. 1) is delivering breathing gas to patient 10 (FIG. 1)), frame member 120 is under tension and is oriented concavely with respect to dial member 160. In other words, frame member 120 is under tension and partially wraps around dial member 160 so that dial member 160 is generally internal with respect to frame member 120.

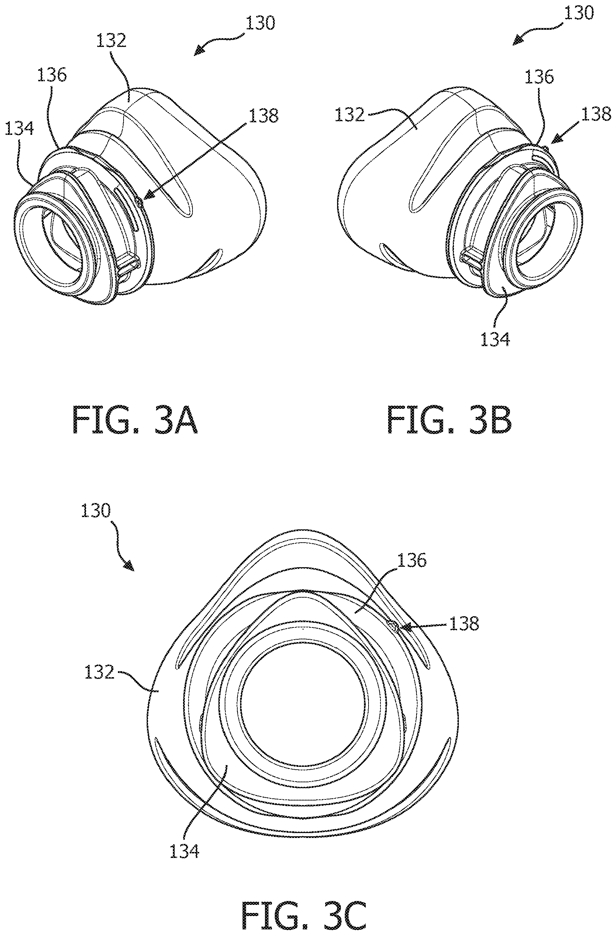

FIGS. 3A-3C show different views of body member 130. Body member 130 includes a cushion portion 132 and a mounting portion 134. Cushion portion 132 is softer than mounting portion 134, and in operation cushion portion 132 engages patient 10 (FIG. 1). Frame member 120 is coupled to mounting portion 134 because mounting portion 134 is relatively rigid. Body member 130 further has a generally annular-shaped raised rim 136 located in mounting portion 134. Raised rim 136 includes a protrusion 138, the function of which will be discussed below.

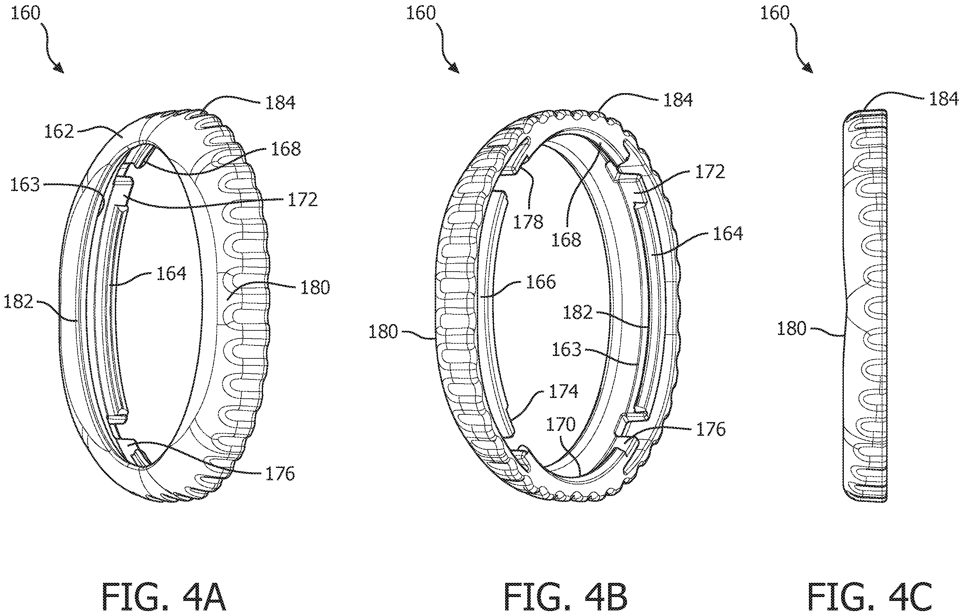

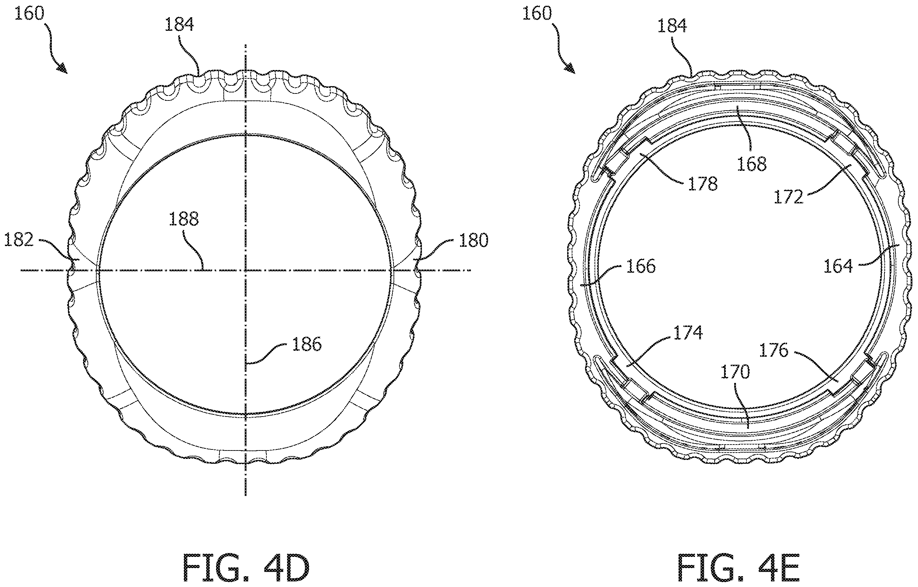

FIGS. 4A-4E show different views of dial member 160. Dial member 160 includes an external lip 162, a number of internal lips 164,166,168,170, and has a grooved region 163 located between external lip 162 and internal lips 164,166,168,170. Located between each adjacent pair of internal lips 164,166,168,170 is a corresponding internal recess 172,174,176, 178. Internal recess 172 is located opposite internal recess 174, and internal recess 176 is located opposite internal recess 178. Furthermore, internal recesses 172,174 are each located between internal recesses 176,178. Dial member 160 also has a pair of opposing recessed portions 180,182. During pressure support therapy, dial member 160 is configured to be rotated with respect to body member 130. To aide this rotation, dial member 160 advantageously includes a corrugated peripheral edge 184 that is a relatively rough portion of dial member 160 for patient 10 (FIG. 1) to grasp.

Raised rim 136 (FIGS. 3A-3C) of body member 130 is located in grooved region 163 in order to allow dial member 160 to be maintained on patient interface device 100. When leaks develop between patient 10 and body member 130 (e.g., without limitation, leaks due to movement in bed or relaxation of the muscles, such as for example when patient 10 is asleep and using pressure support system 2), patient 10 simply needs to rotate dial member 160 with respect to body member 130. When this is done, raised rim 136 slides in grooved region 163, and adjustment assembly 110 moves from a first position to a second position. Moreover, because raised rim 136 is located in mounting portion 134, raised rim is advantageously a relatively rigid structure on which dial member 160 can rotate. Although the disclosed concept has been described in association with raised rim 136 of body member 130 sliding in grooved region 163 of dial member 160, it is within the scope of the disclosed concept to have any suitable alternative configuration (not shown) which allows a suitable alternative dial member (not shown) to perform the desired function of rotating with respect to a suitable alternative body member (not shown).

The first position is a more relaxed position and the second position is a tighter position. Stated differently, cushion portion 132 exerts a greater force on patient 10 when adjustment assembly 110 is in the second position than when adjustment assembly 110 is in the first position. Thus, leaks between patient 10 and cushion portion 132 that are present when adjustment assembly 110 is in the first position are more likely not to be present when adjustment assembly 110 is in the second position. More specifically, frame member 120 exerts a force on dial member 160 when frame member 120 is under tension and oriented concavely with respect to dial member 160 (i.e., during pressure support therapy). When adjustment assembly 110 moves from the first position to the second position, the force exerted by frame member 120 on dial member 160 increases. Because dial member 160 is maintained on body member 130, this results in cushion portion 132 being pushed tighter against (i.e., exerting a greater force on) the face of patient 10, advantageously minimizing leaks. Because headgear straps 8 (FIG. 1) do not need to be adjusted, adjustment assembly 110 provides a relatively fast and easy mechanism to minimize leaks without changing settings.

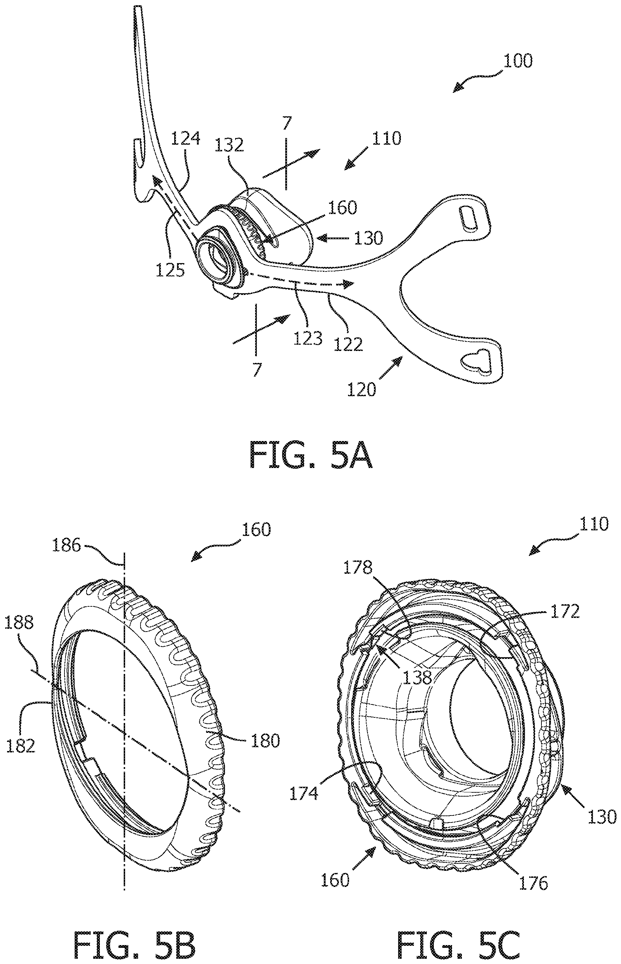

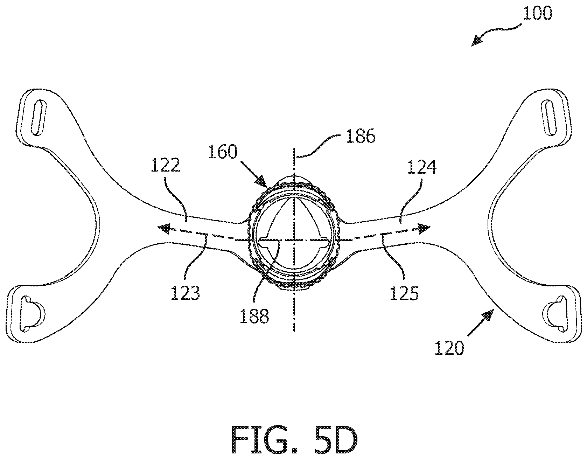

FIG. 5A shows adjustment assembly 110 in the first position. As shown, dial member 160 is substantially located between frame member 120 and cushion portion 132. Dial member 160 is also oriented concavely with respect to cushion portion 132, which allows dial member 160 to slide more easily on frame member 120. Frame member 120 includes a number of arm portions 122,124. Arm portion 122 extends from proximate body member 130 in a first direction 123, and arm portion 124 extends from proximate body member 130 in a second direction 125 generally opposite direction 123. As shown in FIG. 5B, dial member 160 has a major axis 186 and a minor axis 188 generally perpendicular to major axis 186. Furthermore, minor axis 188 extends through recessed portions 180,182. When adjustment assembly 110 is in the first position, minor axis 188 is aligned with directions 123,125. To illustrate, reference is made to FIG. 5D, which shows a back elevation view of patient interface device 100 without body member 130. As shown, minor axis 188 intersects arm portions 122,124 and is aligned with (i.e., generally parallel with respect to) directions 123,125. By contrast, major axis 186 does not intersect either of arm portions 122,124 and is not aligned with directions 123,125.

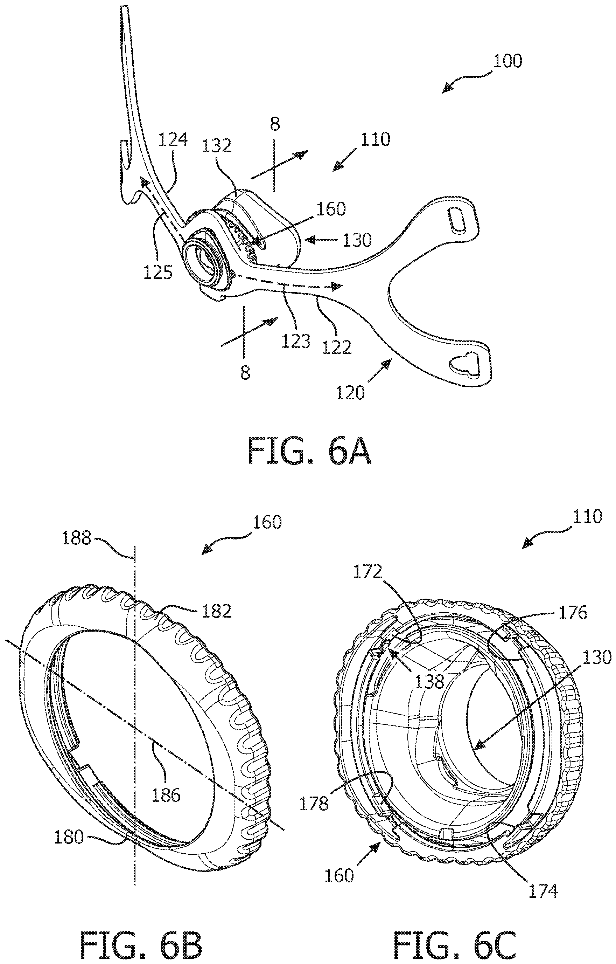

Referring to FIG. 5C, when protrusion 138 is located in internal recess 178, adjustment assembly 110 is in the first position. It will be appreciated that when dial member 160 is rotated 180 degrees, adjustment assembly 110 would still be in the first position, in which case protrusion 138 would be located in internal recess 176. When patient 10 desires to tighten patient interface device 100 (i.e., to minimize leaks), patient 10 simply needs to rotate dial member 160 so that adjustment assembly 110 moves from the first position (FIGS. 5A-5D) to the second position (FIGS. 6A-6D). This would require rotating dial member 160 clockwise or counterclockwise 90 degrees.

By having recessed portions 180,182, dial member 160 is advantageously able to slide on frame member 120 more easily. Specifically, when adjustment assembly 110 is in the first position (FIGS. 5A-5D), arm portions 122,124 engage respective recessed portions 180,182, and when adjustment assembly 110 is in the second position (FIGS. 6A-6D), arm portions 122,124 do not engage recessed portions 180,182. As adjustment assembly 110 moves from the first position (FIGS. 5A-5D) to the second position (FIGS. 6A-6D), recessed portions 180,182 allow for a relatively smooth transition. This is necessary because the force exerted by frame member 120 on dial member 160 increases as adjustment assembly 110 moves from the first position (FIGS. 5A-5D) to the second position (FIGS. 6A-6D), and so without recessed portions 180,182, frictional forces would make rotation of dial member 160 significantly more difficult.

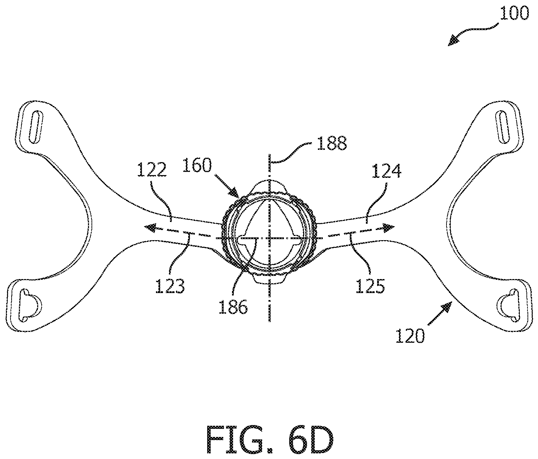

As shown in FIG. 6A, dial member 160 has been rotated 90 degrees from its position when adjustment assembly 110 was in the first position. When adjustment assembly 110 is in this second position, major axis 186 is aligned with directions 123,125 and minor axis 188 is not aligned with directions 123,125. To illustrate, reference is made to FIG. 6D, which shows a back elevation view of patient interface device 100 without body member 130. As shown, major axis 186 intersects arm portions 122,124 and is aligned with (i.e., generally parallel with respect to) directions 123,125. By contrast, minor axis 188 does not intersect either of arm portions 122,124 and is not aligned with directions 123,125. Referring to FIG. 6C, when protrusion 138 is located in internal recess 172, adjustment assembly 110 is in the second position. It will be appreciated that when dial member 160 is rotated 180 degrees, adjustment assembly 110 would still be in the second position, however protrusion 138 would be located in internal recess 174.

Protrusion 138 and internal recesses 172,174,176,178 advantageously provide a mechanism by which patient 10 can quickly and easily determine which position adjustment assembly 110 is in. For example and without limitation, during use, when adjustment assembly 110 is in the first position, protrusion 138 is located in either internal recess 176 or internal recess 178. Because internal recesses 176,178 are opposite each other, and because internal recesses 172,174 are located between internal recesses 176,178, patient 10 would only need to rotate dial member 160 one turn in order to move adjustment assembly 110 to the second position.

More specifically, when dial member 160 is rotated, it makes a "clicking" sound, which is caused by protrusion 138 entering a respective one of internal recesses 172,174,176,178. When adjustment assembly 110 moves from the first position to the second position, patient 10 rotates dial member 160 either clockwise or counterclockwise. As a result, protrusion 138 exits a respective one of internal recesses 176,178, and by detecting a first "click," patient 10 can reliably determine that dial member 160 has rotated 90 degrees (i.e., detecting that protrusion 138 has moved to one of internal recesses 172,174). A second "click" would indicate that adjustment assembly 110 has returned to the first position. Similarly, when adjust assembly 110 is in the second position, protrusion 138 is located in either internal recess 172 or internal recess 174. By rotating dial member 160 either clockwise or counterclockwise, detecting a single "click" provides a quick and reliable mechanism by which patient 10 can determine that adjustment assembly 110 has moved to the first position.

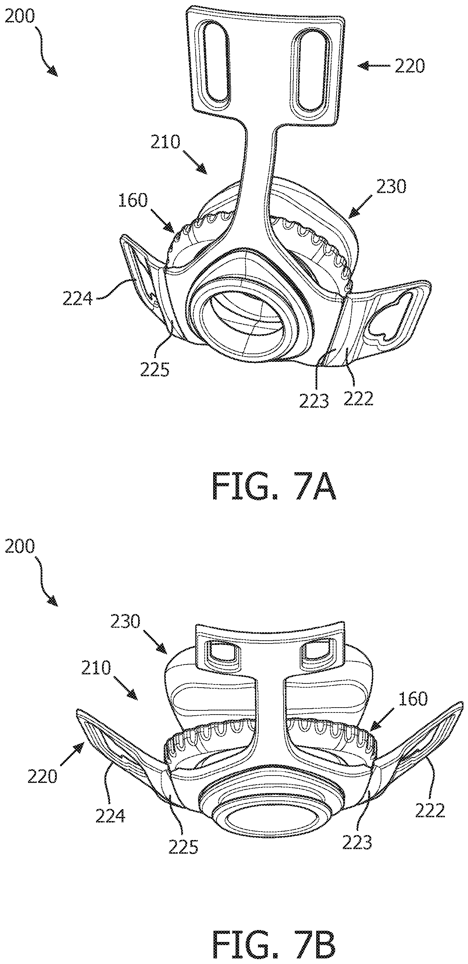

FIGS. 7A and 7B show another patient interface device 200 that may be used in pressure support system 2 (FIG. 1) instead of patient interface device 100. Patient interface device 200 includes an adjustment assembly 210 and a frame member 220. Adjustment assembly 210 includes dial member 160 and a body member 230. Body member 230 is substantially the same as body member 130 (described hereinabove) . Frame member 220 is made of a relatively rigid material (e.g., without limitation, plastic). As shown, frame member 220 includes a pair of opposing arm portions 222,224, each extending from proximate body member 230. It will be appreciated that when frame member 220 is under tension and oriented concavely with respect to dial member 160, as shown, adjustment assembly 210 is structured to move between a first position and a second position in substantially the same manner as adjustment assembly 110 (described above in association with FIGS. 1-6D), thus providing substantially the same benefits as adjustment assembly 110.

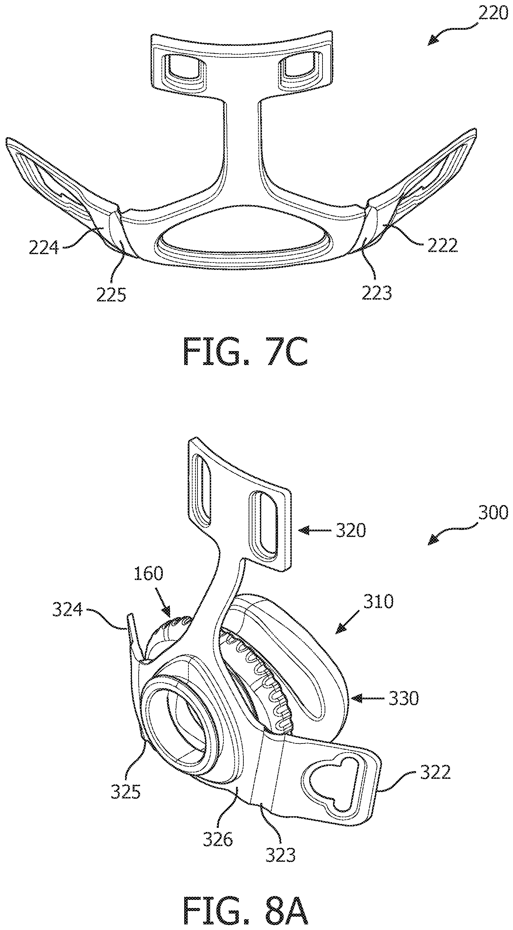

Additionally, because frame member 220 is relatively rigid, arm portions 222,224 each include a respective living hinge 223,225. As shown in FIG. 7C, living hinges 223,225 are generally thinned regions in arm portions 222,224, which advantageously allow frame member 220 to flex as adjustment assembly 210 moves between the first and second positions. In other words, living hinges 223,225 each have a respective thickness that is less than the thickness of a corresponding one of arm portions 222,224. Thus, when adjustment assembly 210 moves from the first position (not shown) to the second position (FIGS. 7A and 7B), arm portion 222 pivots about living hinge 223, and arm portion 224 pivots about living hinge 225.

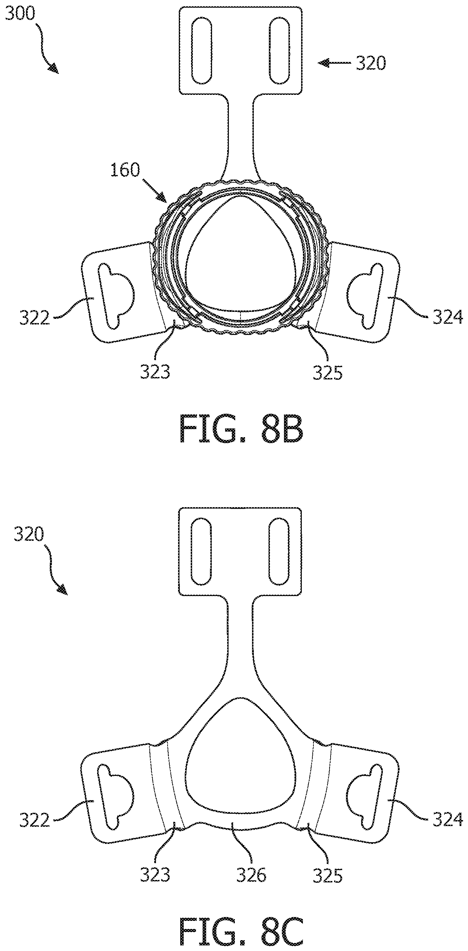

FIGS. 8A and 8B show another patient interface device 300 that may be used in pressure support system 2 (FIG. 1) instead of patient interface device 100. Patient interface device 300 includes an adjustment assembly 310 and a frame member 320. Adjustment assembly 310 includes dial member 160 and a body member 330. Body member 330 is substantially the same as body members 130,230 (described hereinabove). As shown in FIG. 8C, frame member 320 includes a pair of arm portions 322,324, a pair of soft hinges 323,325, and a base portion 326. Soft hinge 323 connects arm portion 322 to base portion 326, and soft hinge 325 connects arm portion 324 to base portion 326. Arm portions 322,324 and base portion 326 are made of a relatively rigid material (e.g., plastic) and soft hinges 323,325 are made of a relatively soft material (e.g., silicone). Soft hinge 323 is bonded (e.g., overmolded) to arm portion 322 and base portion 326, and soft hinge 325 is bonded (e.g., overmolded) to arm portion 324 and base portion 326.

It will be appreciated that when frame member 320 is under tension and oriented concavely with respect to dial member 160, as shown, adjustment assembly 310 is structured to move between a first position and a second position in substantially the same manner as adjustment assemblies 110,210 (described above in association with FIGS. 1-6D, and 7A-7C, respectively), thus providing substantially the same benefits as adjustment assemblies 110,210. Additionally, soft hinges 323,325 advantageously allow frame member 320 to flex as adjustment assembly 310 moves between the first and second positions. In other words, because soft hinges 323,325 are softer than arm portions 322,324 and base portion 326, when adjustment assembly 310 moves from the first position (not shown) to the second position (FIGS. 8A and 8B), arm portion 322 pivots about soft hinge 323, and arm portion 324 pivots about soft hinge 325.

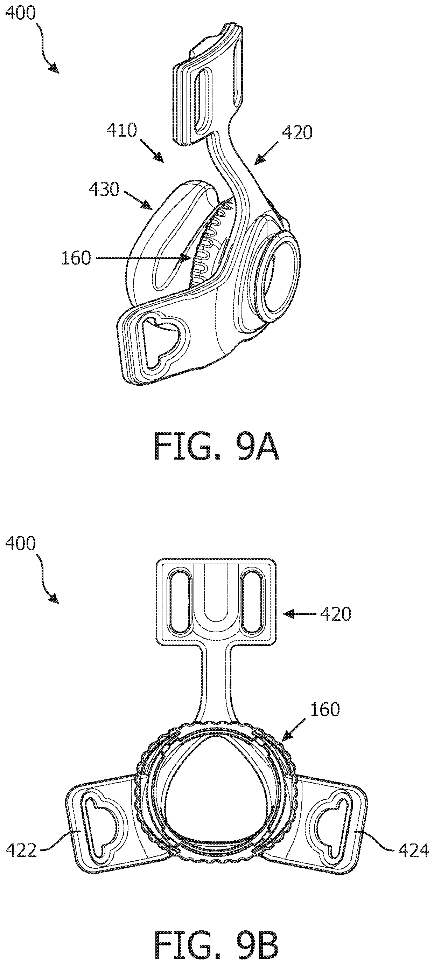

FIGS. 9A and 9B show another patient interface device 400 that may be used in pressure support system 2 (FIG. 1) instead of patient interface device 100. Patient interface device 400 includes an adjustment assembly 410 and a frame member 420. Adjustment assembly 410 includes dial member 160 and a body member 430. Body member 430 is substantially the same as body members 130,230,330 (described hereinabove). Additionally, frame member 420, which includes arm portions 422,424, is made of a fabric material. It will be appreciated that when frame member 420 is under tension and oriented concavely with respect to the dial member 160, as shown, adjustment assembly 410 is structured to move between a first position (not shown) and a second position (FIGS. 9A and 9B) in substantially the same manner as adjustment assemblies 110,210,310 (described above in association with FIGS. 1-6D, 7A-7C, and 8A-8C, respectively), thus providing substantially the same benefits as adjustment assemblies 110,210,310. Additionally, because frame member 420 is made of a fabric material, arm portions 422,424 are advantageously allowed to flex as adjustment assembly 410 moves from the first position (not shown) to the second position (FIGS. 9A and 9B).

Accordingly, it will be appreciated that the disclosed concept provides for an improved (e.g., without limitation, more efficient, easier to adjust) patient interface device 100,200,300,400 and pressure support system 2 therefor, which among other benefits, quickly and reliably allows patient 10 to minimize leaks between cushion portion 132 and patient 10. As a result, adjustment settings, such as settings between frame member 120,220,320,420 and headgear straps 8, advantageously do not need to be adjusted in order for a patient to minimize leaks resulting from relaxing of the muscles during pressure support therapy.

While specific embodiments of the disclosed concept have been described in detail, it will be appreciated by those skilled in the art that various modifications and alternatives to those details could be developed in light of the overall teachings of the disclosure. Accordingly, the particular arrangements disclosed are meant to be illustrative only and not limiting as to the scope of the disclosed concept which is to be given the full breadth of the claims appended hereto.

## Claims

1. A patient interface device (100, 200, 300, 400) for a pressure support system, the pressure support system comprising a fluid coupling conduit and a gas flow generator coupled to the fluid coupling conduit, the gas flow generator being structured to produce a flow of breathing gas for a patient, the patient interface device comprising:
(a) a frame member (120, 220, 320, 420) structured to be secured to the patient; the frame member having a first arm portion (122) and a second arm portion (124); wherein the first arm portion extends from proximate the body member in a first direction; wherein the second arm portion extends from proximate the body member in a second direction generally opposite the first direction and
(b) an adjustment assembly (110, 210, 310, 410) comprising:
(1) a body member (130, 230, 330, 430) coupled to the frame member, the body member being structured to be fluidly coupled to the fluid coupling conduit, and
(2) a dial member (160) coupled to each of the frame member and the body member, wherein the dial member has a first recessed portion and a second recessed portion opposite the first recessed portion, and the frame member (120) partially wraps around dial member (160) so that dial member is generally internal with respect to frame member (120), and the frame member (120) is under tension in operation,
wherein the adjustment assembly is structured to move between a first position and a second position, wherein the frame member exerts a force on the dial member, and wherein, when the adjustment assembly moves from the first position to the second position, the force increases, and wherein when adjustment assembly (110, 210, 310, 410) is in the first position, first and second arm portions (122,124) engage respective recessed portions (180,182) and when adjustment assembly (110) is in the second position, first and second arm portions (122,124) do not engage recessed portions (180,182).

2. The patient interface device of claim 1, wherein the body member extends through each of the frame member and the dial member, and wherein, when the adjustment assembly moves from the first position to the second position, the dial member rotates with respect to the body member.

3. The patient interface device of claim 2, wherein the body member has a generally annular-shaped raised rim; wherein the dial member comprises an external lip and a number of internal lips, wherein the dial member has a grooved region disposed between the external lip and the number of internal lips; and wherein the raised rim is disposed in the grooved region.

4. The patient interface device of claim 3, wherein the raised rim comprises a protrusion; wherein the dial member has a first internal recess, a second internal recess, a third internal recess, and a fourth internal recess; wherein each of the internal recesses is disposed between a corresponding pair of the internal lips; wherein the first internal recess is disposed opposite the second internal recess; wherein the third internal recess is disposed opposite the fourth internal recess; wherein each of the first and second internal recesses is disposed between the third internal recess and the fourth internal recess; wherein, when the protrusion is disposed in the first internal recess, the adjustment assembly is in the first position; wherein, when the protrusion is disposed in the second internal recess, the adjustment assembly is in the first position; wherein, when the protrusion is disposed in the third internal recess, the adjustment assembly is in the second position; and wherein, when the protrusion is disposed in the fourth internal recess, the adjustment assembly is in the second position.

5. The patient interface device of claim 3, wherein the body member further has a cushion portion and a mounting portion, wherein the raised rim is disposed in the mounting portion, and wherein the cushion portion is softer than the mounting portion.

6. The patient interface device of claim 5, wherein the dial member is oriented concavely with respect to the cushion portion.

7. The patient interface device of claim 5, wherein the dial member is substantially disposed between the frame member and the cushion portion.

8. The patient interface device of claim 1, wherein the dial member comprises a corrugated peripheral edge.

9. The patient interface device of claim 1, wherein the first arm portion has a first living hinge; wherein the second arm portion has a second living hinge; wherein, when the adjustment assembly moves from the first position to the second position, the first arm portion pivots about the first living hinge; and wherein, when the adjustment assembly moves from the first position to the second position, the second arm portion pivots about the second living hinge.

10. The patient interface device of claim 1, wherein the frame member comprises a first soft hinge, a second soft hinge, and a base portion; wherein the first soft hinge connects the first arm portion to the base portion; wherein the second soft hinge connects the second arm portion to the base portion; wherein each of the first soft hinge and the second soft hinge is softer than each of the first arm portion, the second arm portion, and the base portion; wherein, when the adjustment assembly moves from the first position to the second position, the first arm portion pivots about the first soft hinge; and wherein, when the adjustment assembly moves from the first position to the second position, the second arm portion pivots about the second soft hinge.

11. The patient interface device of claim 1, wherein the frame member is made of a fabric material.

12. A pressure support system (2) comprising:
(a) a fluid coupling conduit (6);
(b) a gas flow generator (4) coupled to the fluid coupling conduit, the gas flow generator being structured to produce a flow of breathing gas for a patient (10); and
(c) a patient interface device (100,200,300,400) device according to any one of claims 1-11.

## Patentansprüche

1. Patientenschnittstellenvorrichtung (100, 200, 300, 400) für ein Druckunterstützungssystem, das Druckunterstützungssystem umfassend eine Fluidkopplungsleitung und einen Gasstromgenerator, der an die Fluidkopplungsleitung gekoppelt ist, wobei der Gasstromgenerator strukturiert ist, einen Strom Atemgas für einen Patienten zu erzeugen, die Patientenschnittstellenvorrichtung umfassend:
(a) ein Rahmenelement (120, 220, 320, 420), das strukturiert ist, an dem Patienten gesichert zu werden; wobei das Rahmenelement einen ersten Armabschnitt (122) und einen zweiten Armabschnitt (124) aufweist; wobei sich der erste Armabschnitt aus der Nähe des Körperelements in eine erste Richtung erstreckt, wobei sich der zweite Armabschnitt aus der Nähe des Körperelements in eine zweite Richtung erstreckt, die im Allgemeinen der ersten Richtung entgegengesetzt ist, und
(b) eine Einstellungsanordnung (110, 210, 310, 410), umfassend:
(1) ein Körperelement (130, 230, 330, 430), das an das Rahmenelement gekoppelt ist, wobei das Körperelement strukturiert ist, strömungstechnisch an die Fluidkopplungsleitung gekoppelt zu sein, und
(2) ein Drehscheibenelement (160), das an jedes von dem Rahmenelement und dem Körperelement gekoppelt ist, wobei das Drehscheibenelement einen ersten vertieften Abschnitt und einen zweiten vertieften Abschnitt gegenüber dem ersten vertieften Abschnitt aufweist und das Rahmenelement (120) teilweise um das Drehscheibenelement (160) herumgewickelt ist, sodass das Drehscheibenelement im Allgemeinen im Inneren in Bezug auf das Rahmenelement (120) liegt, und das Rahmenelement (120) in Betrieb unter Spannung steht, wobei
die Einstellungsanordnung strukturiert ist, sich zwischen einer ersten Position und einer zweiten Position zu bewegen, wobei das Rahmenelement eine Kraft auf das Drehscheibenelement ausübt, und wobei, wenn sich die Einstellungsanordnung aus der ersten Position in die zweite Position bewegt, die Kraft zunimmt, und wobei, wenn sich die Einstellungsanordnung (110, 210, 310, 410) in der ersten Position befindet, der erste und zweite Armabschnitt (122, 124) mit dem entsprechenden vertieften Abschnitt (180, 182) in Eingriff sind, und wenn sich die Einstellungsanordnung (110) in der zweiten Position befindet, der erste und zweite Armabschnitt (122, 124) nicht mit dem entsprechenden vertieften Abschnitt (180, 182) in Eingriff sind.

2. Patientenschnittstellenvorrichtung nach Anspruch 1, wobei sich das Körperelement durch jedes von dem Rahmenelement und dem Drehscheibenelement erstreckt und wobei, wenn sich die Einstellungsanordnung aus der ersten Position in die zweite Position bewegt, das Drehscheibenelement sich in Bezug auf das Körperelement dreht.

3. Patientenschnittstellenvorrichtung nach Anspruch 2, wobei das Körperelement einen im Allgemeinen ringförmigen erhabenen Rand aufweist, wobei das Drehscheibenelement eine äußere Lippe und eine Reihe von inneren Lippen umfasst, wobei das Drehscheibenelement einen gerillten Bereich aufweist, der zwischen der äußeren Lippe und der Reihe von inneren Lippen angeordnet ist; und wobei der erhabene Rand in dem gerillten Bereich angeordnet ist.

4. Patientenschnittstellenvorrichtung nach Anspruch 3, wobei der erhabene Rand einen Vorsprung umfasst; wobei das Drehscheibenelement eine erste innere Vertiefung, eine zweite innere Vertiefung, eine dritte innere Vertiefung und eine vierte innere Vertiefung aufweist, wobei jede der inneren Vertiefungen zwischen einem entsprechenden Paar innerer Lippen angeordnet ist, wobei die erste innere Vertiefung gegenüber der zweiten inneren Vertiefung angeordnet ist; wobei die dritte innere Vertiefung gegenüber der vierten inneren Vertiefung angeordnet ist; wobei jede der ersten und zweiten inneren Vertiefung zwischen der dritten inneren Vertiefung und der vierten inneren Vertiefung angeordnet ist; wobei, wenn der Vorsprung in der ersten inneren Vertiefung angeordnet ist, die Einstellungsanordnung sich in der ersten Position befindet; wobei, wenn der Vorsprung in der zweiten inneren Vertiefung angeordnet ist, die Einstellungsanordnung sich in der ersten Position befindet; wobei, wenn der Vorsprung in der dritten inneren Vertiefung angeordnet ist, die Einstellungsanordnung sich in der zweiten Position befindet; und wobei, wenn der Vorsprung in der vierten inneren Vertiefung angeordnet ist, die Einstellungsanordnung sich in der zweiten Position befindet.

5. Patientenschnittstellenvorrichtung nach Anspruch 3, wobei das Körperelement weiter einen Dämpfungsabschnitt und einen Montageabschnitt aufweist, wobei der erhabene Rand im Montageabschnitt angeordnet ist und wobei der Dämpfungsabschnitt weicher ist als der Montageabschnitt.

6. Patientenschnittstellenvorrichtung nach Anspruch 5, wobei das Drehscheibenelement konkav in Bezug auf den Dämpfungsabschnitt orientiert ist.

7. Patientenschnittstellenvorrichtung nach Anspruch 5, wobei das Drehscheibenelement im Wesentlichen zwischen dem Rahmenelement und dem Dämpfungsabschnitt angeordnet ist.

8. Patientenschnittstellenvorrichtung nach Anspruch 1, wobei das Drehscheibenelement einen welligen Umfangsrand umfasst.

9. Patientenschnittstellenvorrichtung nach Anspruch 1, wobei der erste Armabschnitt ein erstes bewegliches Scharnier aufweist, wobei der zweite Armabschnitt ein zweites bewegliches Scharnier aufweist; wobei, wenn sich die Einstellungsanordnung aus der ersten Position in die zweite Position bewegt, der erste Armabschnitt um das erste bewegliche Scharnier schwenkt; und wobei, wenn sich die Einstellungsanordnung aus der ersten Position in die zweite Position bewegt, der zweite Armabschnitt um das zweite bewegliche Scharnier schwenkt.

10. Patientenschnittstellenvorrichtung nach Anspruch 1, wobei das Rahmenelement ein erstes weiches Scharnier, ein zweites weiches Scharnier und einen Basisabschnitt umfasst; wobei das erste weiche Scharnier den ersten Armabschnitt mit dem Basisabschnitt verbindet; wobei das zweite weiche Scharnier den zweiten Armabschnitt mit dem Basisabschnitt verbindet; wobei jedes von dem ersten weichen Scharnier und dem zweiten weichen Scharnier weicher ist als jeder von dem ersten Armabschnitt, dem zweiten Armabschnitt und dem Basisabschnitt; wobei, wenn sich die Einstellungsanordnung aus der ersten Position in die zweite Position bewegt, der erste Armabschnitt um das erste weiche Scharnier schwenkt; und wobei, wenn sich die Einstellungsanordnung aus der ersten Position in die zweite Position bewegt, der zweite Armabschnitt um das zweite weiche Scharnier schwenkt.

11. Patientenschnittstellenvorrichtung nach Anspruch 1, wobei das Rahmenelement aus einem Stoffmaterial besteht.

12. Druckunterstützungssystem (2), umfassend:
(a) eine Fluidkopplungsleitung (6);
(b) einen Gasstromgenerator (4), der an die Fluidkopplungsleitung gekoppelt ist, wobei der Gasstromgenerator strukturiert ist, einen Strom Atemgas für einen Patienten (10) zu erzeugen; und
(c) eine Patientenschnittstellenvorrichtung (100, 200, 300, 400) nach einem der Ansprüche 1-11.

## Revendications

1. Dispositif d'interface de patient (100, 200, 300, 400) pour un système support de pression, le système support de pression comprenant un conduit d'accouplement fluidique et un générateur d'écoulement de gaz accouplé au conduit d'accouplement fluidique, le générateur d'écoulement de gaz étant structuré pour produire un écoulement de gaz respiratoire pour un patient, le dispositif d'interface de patient comprenant :
(a) un élément armature (120, 220, 320, 420) structuré pour être fixé au patient ; l'élément armature présentant une première partie bras (122) et une seconde partie bras (124) ; dans lequel la première partie bras s'étend depuis la proximité de l'élément corps dans une première direction ; dans lequel la seconde partie bras s'étend depuis la proximité de l'élément corps dans une seconde direction globalement opposée à la première direction et
(b) un ensemble d'ajustement (110, 210, 310, 410) comprenant :
( 1) un élément corps (130, 230, 330, 430) accouplé à l'élément armature, l'élément corps étant structuré pour être accouplé de manière fluidique au conduit d'accouplement fluidique, et
( 2) un élément cadran (160) accouplé à chacun de l'élément armature et l'élément corps, dans lequel l'élément cadran présente une première partie en creux et une seconde partie en creux opposée à la première partie en creux, et l'élément armature (120) enveloppe partiellement l'élément cadran (160) de sorte que l'élément cadran soit globalement interne par rapport à l'élément armature (120), et l'élément armature (120) subit une tension en fonctionnement,
dans lequel l'ensemble d'ajustement est structuré pour se déplacer entre une première position et une seconde position, dans lequel l'élément armature exerce une force sur l'élément cadran, et dans lequel, lorsque l'ensemble d'ajustement se déplace depuis la première position jusqu'à la seconde position, la force augmente, et dans lequel lorsque l'ensemble d'ajustement (110, 210, 310, 410) est dans la première position, les première et seconde parties bras (122, 124) viennent en prise avec des parties en creux (180, 182) respectives et lorsque l'ensemble d'ajustement (110) est dans la seconde position, les première et seconde parties bras (122, 124) ne viennent pas en prise avec les parties en creux (180, 182).

2. Dispositif d'interface de patient selon la revendication 1, dans lequel l'élément corps s'étend à travers chacun de l'élément armature et l'élément cadran, et dans lequel, lorsque l'ensemble d'ajustement se déplace depuis la première position jusqu'à la seconde position, l'élément cadran tourne par rapport à l'élément corps.

3. Dispositif d'interface de patient selon la revendication 2, dans lequel l'élément corps présente un bord surélevé de forme globalement annulaire ; dans lequel l'élément cadran comprend une lèvre externe et plusieurs lèvres internes, dans lequel l'élément cadran présente une région rainurée disposée entre la lèvre externe et les plusieurs lèvres internes ; et dans lequel le bord surélevé est disposé dans la région rainurée.

4. Dispositif d'interface de patient selon la revendication 3, dans lequel le bord surélevé comprend une saillie ; dans lequel l'élément cadran présente un premier creux interne, un deuxième creux interne, un troisième creux interne et un quatrième creux interne ; dans lequel chacun des creux internes est disposé entre une paire correspondante des lèvres internes ; dans lequel le premier creux interne est disposé à l'opposé du deuxième creux interne ; dans lequel le troisième creux interne est disposé à l'opposé du quatrième creux interne ; dans lequel chacun des premier et deuxième creux internes est disposé entre le troisième creux interne et le quatrième creux interne ; dans lequel, lorsque la saillie est disposée dans le premier creux interne, l'ensemble d'ajustement est dans la première position ; dans lequel, lorsque la saillie est disposée dans le deuxième creux interne, l'ensemble d'ajustement est dans la première position ; dans lequel, lorsque la saillie est disposée dans le troisième creux interne, l'ensemble d'ajustement est dans la seconde position ; et dans lequel, lorsque la saillie est disposée dans le quatrième creux interne, l'ensemble d'ajustement est dans la seconde position.

5. Dispositif d'interface de patient selon la revendication 3, dans lequel l'élément corps présente en outre une partie coussin et une partie de montage, dans lequel le bord surélevé est disposé dans la partie de montage, et dans lequel la partie coussin est plus molle que la partie de montage.

6. Dispositif d'interface de patient selon la revendication 5, dans lequel l'élément cadran est orienté de manière concave par rapport à la partie coussin.

7. Dispositif d'interface de patient selon la revendication 5, dans lequel l'élément cadran est disposé sensiblement entre l'élément armature et la partie coussin.

8. Dispositif d'interface de patient selon la revendication 1, dans lequel l'élément cadran comprend une bordure périphérique ondulée.

9. Dispositif d'interface de patient selon la revendication 1, dans lequel la première partie bras présente une première charnière vivante ; dans lequel la seconde partie bras présente une seconde charnière vivante ; dans lequel, lorsque l'ensemble d'ajustement se déplace depuis la première position jusqu'à la seconde position, la première partie bras pivote autour de la première charnière vivante ; et dans lequel, lorsque l'ensemble d'ajustement se déplace depuis la première position jusqu'à la seconde position, la seconde partie bras pivote autour de la seconde charnière vivante.

10. Dispositif d'interface de patient selon la revendication 1, dans lequel l'élément armature comprend une première charnière molle, une seconde charnière molle et une partie base ; dans lequel la première charnière molle relie la première partie bras à la partie base ; dans lequel la seconde charnière molle relie la seconde partie bras à la partie base ; dans lequel chacune de la première charnière molle et la seconde charnière molle est plus molle que chacune de la première partie bras, la seconde partie bras et la partie base ; dans lequel, lorsque l'ensemble d'ajustement se déplace depuis la première position jusqu'à la seconde position, la première partie bras pivote autour de la première charnière molle ; et dans lequel, lorsque l'ensemble d'ajustement se déplace depuis la première position jusqu'à la seconde position, la seconde partie bras pivote autour de la seconde charnière molle.

11. Dispositif d'interface de patient selon la revendication 1, dans lequel l'élément armature est constitué d'un matériau en tissu.

12. Système support de pression (2) comprenant :
(a) un conduit d'accouplement fluidique (6) ;
(b) un générateur d'écoulement de gaz (4) accouplé au conduit d'accouplement fluidique, le générateur d'écoulement de gaz étant structuré pour produire un écoulement de gaz respiratoire pour un patient (10) ; et
(c) un dispositif d'interface de patient (100, 200, 300, 400) dispositif selon l'une quelconque des revendications 1 à 11.
